# EUROPEAN PATENT SPECIFICATION

(11) **EP 0 792 154 B1**
(45) Date of publication and mention of the grant of the patent: **16.04.2003**
(21) Application number: 95939282.0
(22) Date of filing: 14.11.1995
(51) Int. Cl.: A61K 33/24, A61K 31/185

(54) **COMPOSITION OF CISPLATIN IN COMBINATION WITH 2,2'-DITHIO-BIS(ETHANESULFONATE) (DIMESNA)**
ZUSAMMENSETZUNG AUS CISPLATIN IN KOMBINATION MIT 2,2'-DITHIO-BIS(ETHANSULFONAT)(DIMESNA)
COMPOSITION DE CISPLATINE COMBINE AVEC DU 2,2'-DITHIO-BIS-(ETHANESULFONATE) (DIMESNA)

(30) Priority: 14.11.1994 US 338379
(43) Date of publication of application: 03.09.1997
(73) Proprietor: BIONUMERIK PHARMACEUTICALS, INC., San Antonio, Texas 78229 (US)
(72) Inventor: HAUSHEER, Frederick, Herman, San Antonio, TX 78209 (US); HARIDAS, Kochat, San Antonio, TX 78240 (US); MURALI, Dhanabalan, San Antonio, TX 78230 (US); REDDY, Dasharatha, Gauravaram, San Antonio, TX 78229 (US)
(74) Representative: Lucas, Brian Ronald
(86) International application number: PCT/EP95/04490
(87) International publication number: WO 96/014852

(56) References cited:
- EP-A- 0 334 083
- EP-A- 0 538 858
- EUR J CANCER, vol. 27, no. 10, 1991 pages 1243-1247, O.R. LEEUWENKAMP ET AL. 'Reaction kinetics of cisplatin and its monoaquated species with the modulating agents (di)mesna and thiosulphate'
- CANCER CHEMOTHER PHARMACOL, vol. 27, no. 2, 1990 pages 111-114, O.R. LEEUWENKAMP ET AL. 'Reaction kinetics of cisplatin and its monoaquated species with the (potential) renal protecting agents (di)mesna and thiosulfate'
- D. SCHMÄHL 'Combination effects in chemical carcinogenesis' 1988 , ED. WEINHEIM, VCH PUBLISHERS see page 153 - page 173 see page 162 - page 166
- ARZNEIM.-FORSCH., vol. 32, no. 5, 1982 pages 486-487, N. BROCK ET AL. 'Verhütung urotoxischer Wirkungen von Cyclophosphamid und Ifosfamid durch Dimesna'
- EUR J CANCER CLIN ONCOL, vol. 17, 1981 pages 1155-1163, N. BROCK ET AL. 'Studies on the urotoxicity of oxazaphosphorine cytostatics and its prevention.' cited in the application
- J CANCER RES CLIN ONCOL, vol. 108, 1984 pages 87-97, N. BROCK ET AL. 'Pharmacokinetics and mechanism of action of detoxifying low-molecular-weight thiols' cited in the application
- CANCER CHEMOTHER PHARMACOL, vol. 18, no. suppl. 2, 1986 pages s1-s9, W. BRADE ET AL. 'Comparative activity of ifosfamide and cyclophosphamide'
- BRITISH J CANCER, vol. 52, 1985 pages 937-939, S.R. KEMPF ET AL. 'Effective prevention of nephrotoxicity of cis-platin (CDDP) by administration of sodium 2-mercaptoethane-sulfonate (MESNA) in rats' cited in the application
- CLIN PHARM, vol. 9, no. 3, 1990 pages 179-191, S.E. SCHOENIKE ET AL. 'Ifosfamide and mesna'

## Description

### FIELD OF THE INVENTION

This invention relates to the use of a compound in the manufacture of a medicament for co-administration with cisplatin to reduce the nephrotoxicity of cisplatin. It also relates to a pharmaceutical composition comprising cisplatin and a compound that is effective to reduce the nephrotoxicity thereof.

### DESCRIPTION OF THE RELATED ART

One of the most important and common dose-limiting toxicities of cisplatin is nephrotoxicity (causing renal damage). In order for cisplatin to react with certain nucleic acid sequences in cellular DNA, it must first undergo chemical conversion to an active species by the displacement of chloride ligands with water to form the mono-aquo or di-aquo species. These reactions are represented by the following equations: ${\text{Pt(NH}}_{\text{3}} {\text{)}}_{\text{2}} {\text{Cl}}_{\text{2}} {\text{+H}}_{\text{2}} {\text{O → [Pt(NH}}_{\text{3}} {\text{)}}_{\text{2}} {\text{Cl(H}}_{\text{2}} {\text{O)]}}^{\text{+}}$${\text{[Pt(NH}}_{\text{3}} {\text{)}}_{\text{2}} {\text{Cl(H}}_{\text{2}} {\text{O)]}}^{\text{+}} {\text{+ H}}_{\text{2}} {\text{O → [Pt(NH}}_{\text{3}} {\text{)}}_{\text{2}} {\text{(H}}_{\text{2}} {\text{O)}}_{\text{2}} {\text{]}}^{\text{++}}$The aquo species of cisplatin are reactive with nucleophilic species, including the imidazole nitrogens on DNA or sulfhydryl groups which are also present in cells forming the renal tubular epithelium in humans.

Cisplatin readily reacts with compounds containing sulfhydryl moieties. Sulfhydryl groups are found in cysteine, glutathione and homocysteine. Metallothionein is a 7 kDa protein containing about 30% cysteine residue. Increased cellular concentrations of metallothionein and glutathione have been correlated with drug resistance to cisplatin therapy. Thus, if the local renal tubular concentration of sulfhydryl groups from 2-mercaptoethanesulfonate could be increased, then cisplatin toxicity might be reduced by the chemical quenching in the renal tubules of the reactive cisplatin aquo species.

Kempf et al., *British J. Cancer,* **52,** 937-939 (1985) describe the use of sodium 2-mercaptoethanesulfonate ("mesna") to prevent the nephrotoxicity of cisplatin in the kidneys of rats. In one series of experiments mesna is administered intraperitoneally to BD IX rats in a dose of 3 mg/kg, which is half the LD₅₀ for cisplatin in that strain. Mesna was administered orally during days 1-4 at a daily dose of 150-1200mg/day and during days 5-12 at a dose of 50-400mg/day, and dose-dependent reductions of toxicity were observed, with kidney damage being unobservable only at the highest dose level. In another series of experiments an adenocarcinoma of the stomach was introduced into BD IX rats that were treated with 3 doses per week of cisplatin in an amount of 1 mg/kg. A first group of animals was treated orally with 400 mg/kg of mesna two hours before each cisplatin dose, and with 200 mg/kg of mesna at four hour intervals up to four times daily, and then with a follow-up treatment of a single daily dose of 400 mg/kg of mesna orally for an additional twelve days. The remaining mice received a balanced electrolyte solution instead of mesna. No difference in tumour inhibition was reported between the two groups. The authors hypothesize that after oral or intravenous administration of mesna, it is converted in the blood to dimesna which does not form a complex with cisplatin or interfere with intravenously administered antiemetics that have to be administered in humans because of the emetic effect of cisplatin. They also hypothesize that the dimesna is eliminated in the kidneys and is largely reduced to dimesna during this process, the dimesna then being available for reaction with cisplatin which mainly damages the renal tubules.

The treatment regime proposed by Kempf *et al.* is complex and impractical. Furthermore, mesna cannot be co-administered with cisplatin, since it would react with it. A problem with which the invention is concerned is to find an alternative to mesna that can be co-administered with cisplatin.

Leeuwenkamp *et al., Chemotherapy and pharmacology, 112-114* (1990) report the results of a theoretical study, based on results reported by others, concerning the kinetics of chemical inactivation of cisplatin in plasma by means of thiosulfate, mesna and diethyldithiocarbamate (DDTC). They conclude that from a kinetic point of view mesna and diethyldithiocarbamate could be useful renal protecting agents, although diethyldithiocarbamate is hampered by severe neurotoxicity. In a further paper, Leeuwenkamp *et al., Eur J. Cancer,* **27,** 1243-1247 (1991) disclose studies of the reaction kinetics between cisplatin and mesna in an un-buffered saline medium at pH 5.3. Because mesna is converted to dimesna in blood, they also investigated the reaction kinetics between cisplatin and dimesna in the same un-buffered saline medium and at the same pH. On the basis of these *in vitro* experiments, the authors suggest that in analogy with thiosulfate protection, urinary (di)mesna concentrations of at least 0.5 mol/l may afford protection against cisplatin-mediated nephrotoxicity, corresponding to an infusion rate of (di)mesna exceeding 100 mg/kg/hr. However, the authors acknowledge a report that mesna does not reduce cisplatin-induced nephrotoxicity in the rat whereas thiosulfate is an effective renal protector, and they ascribe this to the lower reactivity of mesna compared to thiosulfate.

Mesna is widely used to reduce or prevent the risk of hemorrhagic cystitis to the uroepithilium, which causes bleeding in the ureters, bladder and urethra, a condition which is associated with the use of certain oxazaphosphorine anticancer drugs which include cyclophosphamide, ifosfamide and trophosphamide. Dimesna has been tested for the same purpose: see Brock *et al., J. Cancer Res. Clin. Oncol.* **108,** 87-97 1984 and *Eur. J. Cancer Clin. Oncol.* **17,** 1155-1163 (1981). Oxazaphosphorine-induced uroepithilial toxicity is chemically, biochemically, anatomically and pathologically distinct from the renal toxicity which is observed with administration of cisplatin. In the case of oxazaphosphorines, acrolein is produced as a toxic metabolite and mesna undergoes addition to the double bond of acrolein, resulting in a stable thioether adduct which has no damaging effects on the uroepithelium and is excreted in the urine.

### SUMMARY OF THE INVENTION

It has now surprisingly been found that 2,2'-dithio-bis(ethanesulfonate), especially in the form of its sodium salt, dimesna, can be administered substantially simultaneously or near-simultaneously with cisplatin. The sulfonate is not attacked by cysteine and other thiol proteins in the bloodstream and yet is effective to reduce the nephrotoxicity of cisplatin. This is particularly surprising because there is a suspicion that mesna reacts with cysteine in the bloodstream. Increased cysteine elimination in the urine has been reported in association with the administration of mesna to human subjects: see B. Sidau and I.C.Shaw, *J. Chromatography,* **311,** 234-238, (1984). If the metabolism of mesna to dimesna, as stated in the above-mentioned Kempf *et al.* paper is significant, then possibly the dimesna is reacting with cysteine. Alternatively, if mesna is reacting with cysteine, perhaps dimesna is not a significant metabolite of mesna.

One aspect of the invention relates to the use of a pharmaceutically acceptable form of 2,2'-dithio-bis(ethanesulfonate) in the manufacture of a medicament for the reduction of the nephrotoxicity of cisplatin, said medicament being for simultaneous or sequential co-administration with cisplatin to a human patient so that the sulfonate and cisplatin become co-present in the blood of the human patient.

Another aspect of the invention provides a pharmaceutical composition for parenteral administration with cisplatin to a human patient with cancer for potentiating the antitumor activity of and/or reducing the nephrotoxicity of cisplatin, said composition comprising a sterile aqueous solution of 2,2'-dithio-bis-ethane sulfonate, or a pharmaceutically acceptable salt thereof.

A further aspect of the invention provides a pharmaceutical composition for administration to human patients with cancer, comprising cisplatin and a pharmaceutically acceptable form of 2,2'-dithio-bis(ethanesulfonate).

The sulfonate may be in the form of any pharmaceutically acceptable salt, especially the disodium salt (dimesna) or as the free acid or a mixture thereof. Preferably it is in the form of the sodium salt. The preferred form of composition is an aqueous solution, which will normally be a sterile injectable aqueous solution. Such a solution will therefore contain 2,2'-dithio-bis(ethanesulfonate) anions and pharmaceutically acceptable cations, especially Na+ and H+, to provide electrical neutrality.

Use of a composition of the invention will help to ensure that the patient receives the correct dose, will reduce errors in prescribing two drugs and will reduce the amount of additional prophylactic measures needed to reduce nephrotoxicity and avoid iatrogenic related complications (furosemide or hypertonic saline administration - see below).

Compositions of the invention have also been found to potentiate antitumor activity in animal tests. They can also protect against cisplatin-induced myelosuppression or neurotoxicity.

The invention further provides the use of a pharmaceutically acceptable form of 2,2'-dithio-bis(ethanesulfonate), especially the disodium salt (dimesna), in the manufacture of a medicament for administration in combination with cisplatin to a human patient at substantially the same time or sequentially, in either order, especially within 24 hours of one another, whereby the sulfonate and cisplatin become co-present in the blood of the patient. Where patent law permits, notably in Australia, this concept may be expressed alternatively in terms of a method of administering the sulfonate and the cisplatin to the patient at substantially the same time, as set forth above. Thus, this invention further enables the sulfonate and cisplatin to be given sequentially, in either order, especially within 24 hours of one another, or near enough together in time so that the sulfonate enters the bloodstream when the cisplatin is present therein or the cisplatin enters the bloodstream when the sulfonate is present therein, so that the principle of the invention (reduction of nephrotoxicity of cisplatin by the concurrent action therewith *in vivo* of 2,2'-dithio-bis(ethanesulfonate) can have effect.

### Brief Description Of The Drawings

Figures 1 to 3 Values on day 5 *post* treatment with i.v. cisplatin (6 mg/kg) with or without increasing doses of i.v. 2,2'-dithio-bis(ethanesulfonate) (BNP7787):
   - Fig. 1: serum creatinine (mg/dl); Fig. 2: BUN (Blood urea nitrogen)(mg/dl); Fig. 3: WBC (White blood counts)(thousands of counts/mm³)

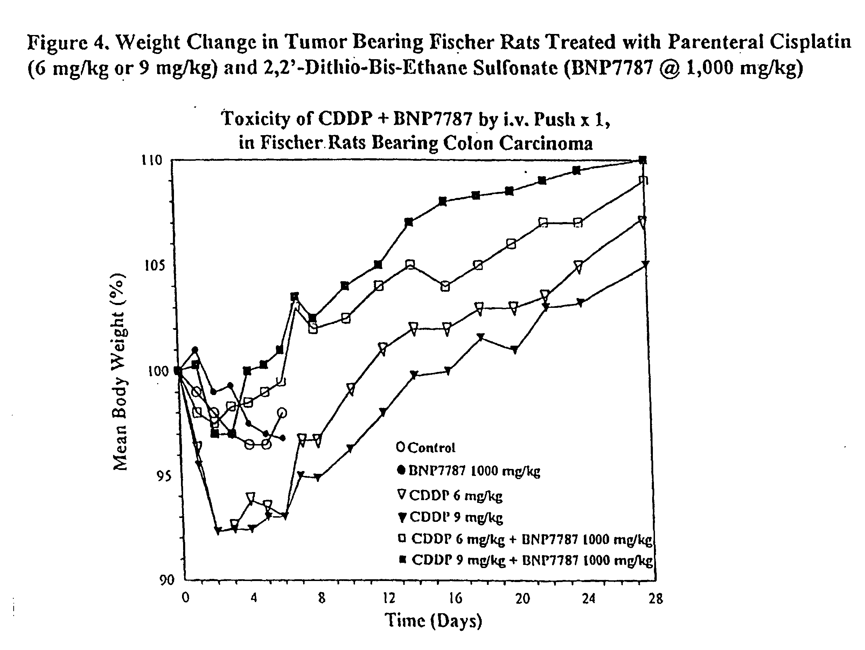

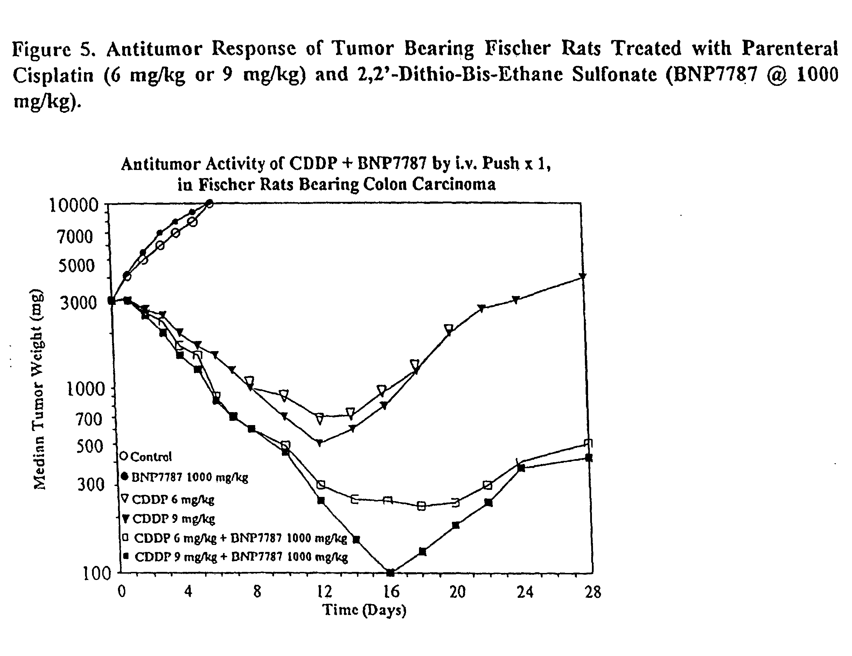

Figures 4 and 5 Responses of colon tumor-bearing Fischer rats injected i.v. with cisplatin and 2,2'-dithio-bis(ethanesulfonate) (BNP7787):
   - Fig. 4: median tumor weight (mg.) vs. days
   - Fig. 5: mean body weight (% starting weight) vs. days

### DESCRIPTION OF THE PREFERRED EMBODIMENTS

The composition of the invention will typically be a solution. It can take any form appropriate to or conventional for formulation of cisplatin, which is not incompatible with the co-presence of the sulfonate or the action of cisplatin. The type of formulation will of course depend on the route of administration, which will normally be parenteral and preferably by injection.

Hereinafter, the invention will be discussed mainly with reference to sterile aqueous solutions of disodium 2,2'-dithio-bis(ethanesulfonate) (dimesna), but the person skilled in the art will readily be able to use the principles of this invention in relation to other formulations of cisplatin and other forms of the sulfonate. Said other forms include the monosodium, monopotassium., sodium-potassium, dipotassium, calcium and magnesium salts of the sulfonate.

Normally the aqueous solution will have a pH less than 7.0 and greater than 1.0, most usually between 2 and 6 and preferably between 4 and 6. This will help to prevent formation of an aquo species of cisplatin. The same pH also serves to prevent the formation of mesna, which could then react with cisplatin species. Preferably hydrochloric acid or phosphoric acid is used to reduce the pH. The solution will preferably contain chloride ion, e.g. provided as NaCl and optionally augmented by use of HCl to reduce the pH, because the stability of cisplatin is proportionally related to the chloride ion concentration of the solution. Also, a high concentration of chloride in the plasma provides conditions which largely favor the maintenance of the unreactive neutral dichloro species of cisplatin. The neutral dichloro cisplatin species can enter cells, such as cancer cells, and because the chloride concentration inside of the cell is low, the conversion of cisplatin dichloro species to mono-aquo or di-aquo species is favored. The aquo cisplatin species is then available to form coordinate chelate crosslinks with certain nucleic acids in cellular DNA.

Cisplatin-induced nephrotoxicity is a clinically important problem and is associated with a decrease in creatinine clearance, elevated creatinine, elevated blood urea nitrogen, elevated uric acid and hypomagnese-mia. Prophylactic measures generally used to try to reduce the risk of this complication include:
a. Parenteral administration of hypertonic (3%) NaCl;
b. Mannitol diuresis;
c. Pre- and/or post-treatment hydration (oral or parenteral);
d. Forced diuresis by the administration of loop diuretics such as furosemide; or
e. Oral or parenteral administration of thiosulfate.

These measures introduce additional risks for patients undergoing treatment. For example, the administration of hypertonic saline (NaCl 30 mg/ml) poses the risk of iatrogenic hypernatremia. Hypernatremia is a life-threatening medical emergency and the administration of hypertonic saline is contra-indicated in patients with elevated serum sodium or patients with congestive heart failure. The use of powerful loop diuretics to increase urine production by the kidney, such as furosemide, increases the iatrogenic risk of hypokalemia, hyponatremia, hypocalcemia, hypovolemia, metabolic alkalosis and hypochloremia. All of these conditions can be life-threatening. They are avoidable by the practice of this invention.

One preferred embodiment of this invention is an injectable, sterile, stable aqueous solution or suspension, which comprises cisplatin, 2,2'-dithio-bis(ethanesulfonate), sodium chloride and hydrochloric or phosphoric acid, especially in a unit dosage form. To preserve sterility, it is stored in a sealed container. This solution is particularly suitable for intravenous injection in human patients with cancer. Preferably the concentration of cisplatin is between about 0.1 mg/ml and about 1.0 mg/ml. Preferably the concentration of 2,2'-dithio-bis(ethanesulfonate) is between 1 mg/ml and its maximum solubility, which, for dimesna, is about 320 mg/ml (measured in either water or saline solution), more preferably between 5 mg/ml and 100 mg/ml (reckoned as the disodium salt). Preferably the concentration of sodium chloride is between 1 and 25 mg/ml, more preferably between 9 and 25 mg/ml and the hydrochloric or phosphoric acid is in a concentration sufficient to provide or maintain the pH in the range of 2 to 6, more preferably 4 to 6. Desirably, the composition also contains a buffer to maintain the pH, especially sodium acetate or phosphate, alone or in combination.

Preferably, the composition of the invention contains mannitol, e.g. where the composition is a solution, usually in a concentration between about 10 and about 25, more preferably between 10 and 15 mg/ml.

The composition of the invention can be prepared by mixing the ingredients in any order.

The same relative proportions of the active principles and of the optional other ingredients can be used to prepare other compositions of the invention. Thus the weight ratio of cisplatin to the sulfonate (reckoned as disodium salt) is preferably from 1:1 to 1:3200 and more preferably from 1:5 to 1: 1000.

When other salts of 2,2'-dithio-bis(ethanesulfonate) are used in place of the disodium salt, the concentration or proportion of the sulfonate will be adjusted according to molarity. 1 g of the disodium salt, dimesna, is equivalent to 4.63 millimoles of 2,2'-dithio-bis(ethanesulfonate) anion.

The 2,2'-dithio-bis(ethanesulfonate)is preferably prepared in lyophilised form, whether for subsequent admixture with cisplatin or for separate administration before or after cisplatin. Aqueous solutions or lyophilates of the sulfonate are stable over a wide pH range, especially 1.5 to 9. Thus, when the sulfonate is administered separately, a pH range of 4 to 9 is preferred. The cisplatin-sulfonate compositions of the invention are also preferably stored as lyophilates. Lyophilates can be reconstituted with sterile water, dextrose (5%) and water, "normal" physiological saline or lactated Ringer's solution. The reconstituted solution is then preferably filtered through a sterile 0.2 micrometre filter, to avoid microaggregation of the drug product.

Administration of cisplatin or a composition of the invention containing it will normally be parenteral, e.g. intravenous, intraarterial, intraperitoneal, subcutaneous, intracavitary or intrapleural. The preferred mode is by injection of an aqueous solution or suspension, preferably intravenously. The sulfonate can be administered separately from the cisplatin parenterally (as above) or orally.

Various regimes of combination administration are possible, e.g. (a) as a one time administration of a composition of the invention, (b) first administering the sulfonate and then the cisplatin within 24 hours or (c) first administering the sulfonate, then the cisplatin one hour later, followed by simultaneous administration of the cisplatin and the sulfonate and, one hour after completion of the cisplatin administration, further sulfonate.

Where the sulfonate is given orally, it can be formulated in any form conventional in the art of pharmaceutical formulation. Solid dosage forms for oral administration include capsules, tablets, pills, powders and granules. They may contain an excipient, e.g. sodium citrate or dicalcium phosphate, or a carrier. Other optional additives may comprise a filler, binder, humectant, disintegrating agent, solution retarder, absorption accelerator, wetting agent, adsorbent, lubricant or buffering agent, as well known in the pharanaceutical formulation art.

Solid compositions of a similar type may also be employed as fillers in soft and hard-filled gelatin capsules using excipients such as lactose or milk sugar as well as high molecular weight polyethylene glycols, and the like.

Preferably the composition is formulated for the delayed release of the 2,2'-dithio-bis(ethanesulfonate) in a certain part of the intestinal tract. Examples of embedding agents for such compositions are polymeric substances and waxes.

Liquid dosage forms for oral administration include physiologically acceptable emulsions, solutions, suspensions, syrups, and elixirs.

The composition of the invention can be given to a patient who has not been treated with an anti-cancer agent (untreated) or to a patient who has previously been treated or exposed to anti-cancer agent(s). Also, it can be administered to patients with cancer in combination with another anti-cancer agent or agents, especially 5-FU, bleomycin, VP-16 (etoposide), cyclophosphamide, ifosfamide, leucovorin, methotrexate, or vinblastine.

The following non-limiting Examples illustrate the invention.The vials referred to are "amber vials", which protect the cisplatin from exposure to fluorescent light.

### EXAMPLE 1

### (a)Preparation of 2,2'-dithio-bis(ethanesulfonate)

Disodium 2,2'-dithio-bis-(ethanesulfonate) was prepared by oxidizing 2-mercaptoethanesulfonate in water with an equimolar amount of iodine as previously reported by L. Lamaire and M. Reiger, J. Org. Chem. 26, 1330-1, (1961).

### (b)Stability of 2,2'-Dithio-Bis-Ethane Sulfonate

50 mg. of the sulfonate thus prepared were dissolved in 1 ml. of water and the pH of the solution adjusted to 1.5, 2.0, 3.0, 4.0, 5.0 and 6.0 by adding 1N hydrochloric acid in water or the pH adjusted to 8.0, 9.0, 10.0 or 11.0 by adding 1 N sodium hydroxide in water. The solution was then stirred for 24 hours at room temperature, the water was removed at reduced pressure and the residue dissolved in spectral grade D₂O. The proton NMR spectrum gave only peaks corresponding to the starting material.

Heating the pH 1.5 solution to 100 deg. C for 10 minutes gave no change in the proton NMR spectrum.

Note that mesna and dimesna show distinctive peaks, whereby degradation of dimesna to mesna could be monitored. The proton NMR spectrum of mesna provided multiplets at 2.88δ and 3.19δ, dimesna at 3.09δ.

### (c) Preparation of a stable solution of cisplatin and 2,2'-dithio-bis(ethanesulfonate)

Pure hydrochloric acid (99.999%) was added to a sterile, injectable, aqueous 0.9% w/v solution of sodium chloride (US Pharmacopeia grade), to give a pH in the range 2.0 to 6.0. 1 mg./ml. of the above NaCl solution of pure cisplatin was added and allowed to completely dissolve by agitation (1500-2500 rpm) at room temperature, for approximately 60 to 90 minutes in the dark. Then, 15 mg of dimesna, prepared above, per ml. of solution were added and the mixture agitated until complete dissolution occurred. The final pH was adjusted to within the range pH 2.0 to 6.0 by adding further pure hydrochloric acid. The solution was sterilized by filtration through a sterile 0.2 micrometre filter (obtained from VWR Scientific) and stored in sterile injection vials. Each vial contained approximately 0.9 mg of cisplatin and 14.3 mg of 2,2'-dithio-bis(ethanesulfonate) per ml of solution.

### EXAMPLE 2

To a sterile injectable aqueous solution of 0.9% w/v sodium chloride (USP grade) were added 15 mg/ml of NaCl solution of dimesna, which was allowed to dissolve completely by agitation (1500-2500 rpm) at room temperature, for 5-10 minutes. The pH of the solution was adjusted to within the range 2.0 to 6.0 by adding pure (99.999%) hydrochloric acid. 1 mg/ml of dimesna solution of pure (99.999%) cisplatin was added and the mixture agitated in the dark until complete dissolution occurred. The remaining steps were as in Example 1(c), giving a solution of the same approximate composition.

### EXAMPLE 3

Example 2 was repeated except that to a 15 mg/ml sterile aqueous solution of dimesna were added 9 mg/ml of dimesna solution of NaCl crystals. Each vial contained approximately 1.0 mg of cisplatin and 14.3 mg of dimesna per ml. of injection solution.

### EXAMPLE 4

Example 1(c) was repeated except that 0.5 mg./ml. of cisplatin and 30 mg./ml. of dimesna were used. Each vial contained 0.5 mg of cisplatin and 30.0 mg of dimesna per ml of injection solution.

### EXAMPLE 5

Example 1(c) was repeated except that USP grade potassium chloride crystals were dissolved in the acidic NaCl solution to give a concentration of 0.1% w/v KCl and also that 30 mg/ml dimesna were used. Each vial contained 1.0 mg of cisplatin and 30.0 mg of dimesna per ml of injection solution.

### EXAMPLE 6

Example 1(c) was repeated except that pure mannitol (99+% purity, purchased from Aldrich Chemical Company) was dissolved in the NaCl solution, to give a concentration of 1.0% w/v mannitol, and also that 30 mg/ml of dimesna were used. Each vial contained approximately 1.0 mg of cisplatin and 30.0 mg of dimesna per ml of injection solution.

### EXAMPLE 7

Example 1(c) was repeated except that the unfiltered, acidified NaCl solution containing cisplatin and 2,2'-dithio-bis(ethanesulfonate) was lyophilized using commercially available equipment. The lyophilate can be stored at room temperature in amber vials, which are shielded from light for 6 months to one year until needed for administration to a patient. It can be reconstituted with sterile water (USP), if necessary reacidified to a pH of 2.0 to 6.0 with hydrochloric or phosphoric acid, and the solution passed through a sterile 0.2 micrometre filter.

### EXAMPLE 8

### In Vivo Demonstration of Protection by 2,2'-Dithio-Bis-(Ethanesulfonate) (BNP7787) Against Cisplatin-induced Nephrotoxicity and Myelosuppression in Fischer Rats.

This Example shows the in vivo protective effects of 2,2'-dithio-bis(ethanesulfonate) (= BNP7787) administered at 1,000 mg/kg by a single i.v. injection in Fischer rats (150 - 200g) receiving a nephrotoxic dose of cisplatin (6 mg/kg i.v. single injection) when the two drugs are administered concurrently (substantially contemporaneously).

Under Good Laboratory Practice conditions, Fischer rats (10 per treatment group) were treated as follows:
Group 1: no treatment
Group 2: normal physiological saline
Group 3: 1000 mg/kg of BNP7787
Group 4: 6 mg/kg cisplatin
Group 5: 6 mg/kg cisplatin and 37 mg/kg BNP7787
Group 6: 6 mg/kg cisplatin and 111 mg/kg BNP 7787
Group 7: 6 mg/kg cisplatin and 333 mg/kg BNP 7787
Group 8: 6 mg/kg cisplatin and 1000 mg/kg BNP 7787

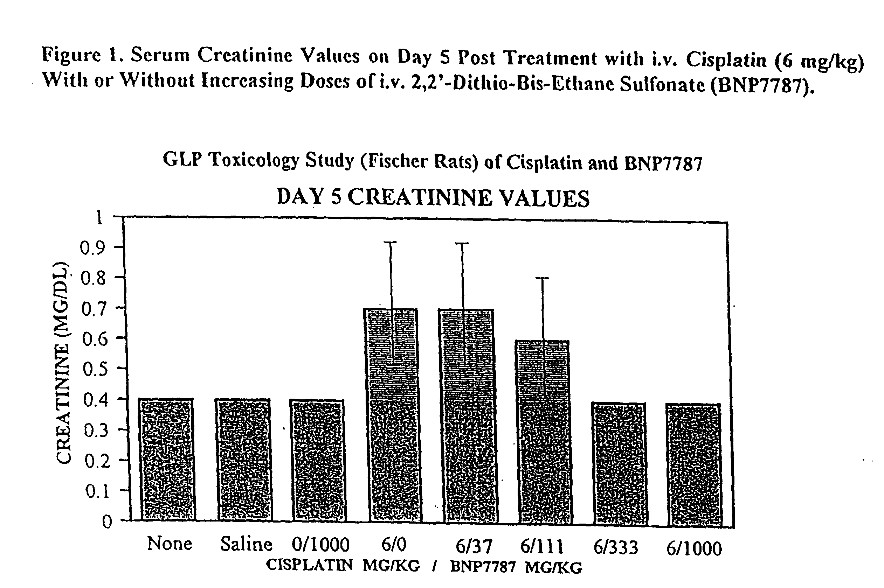

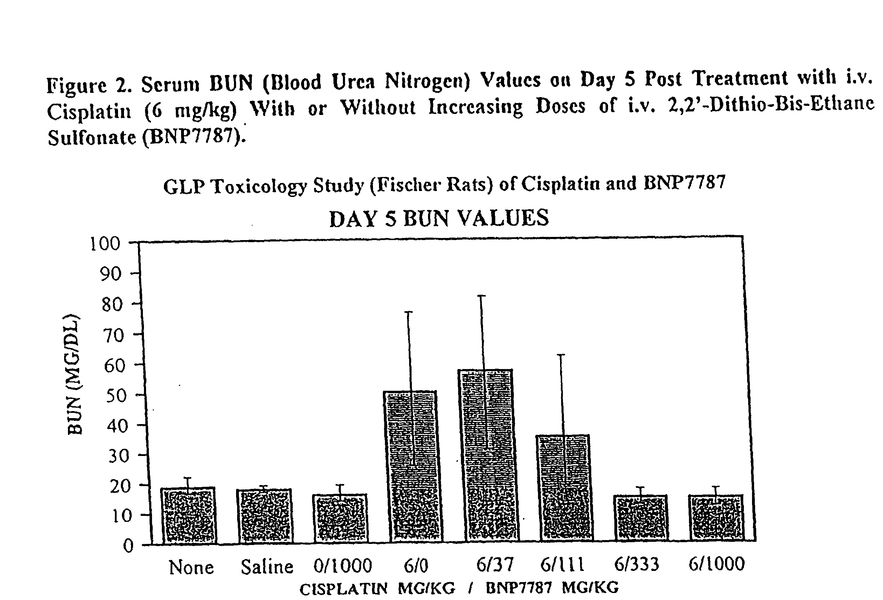

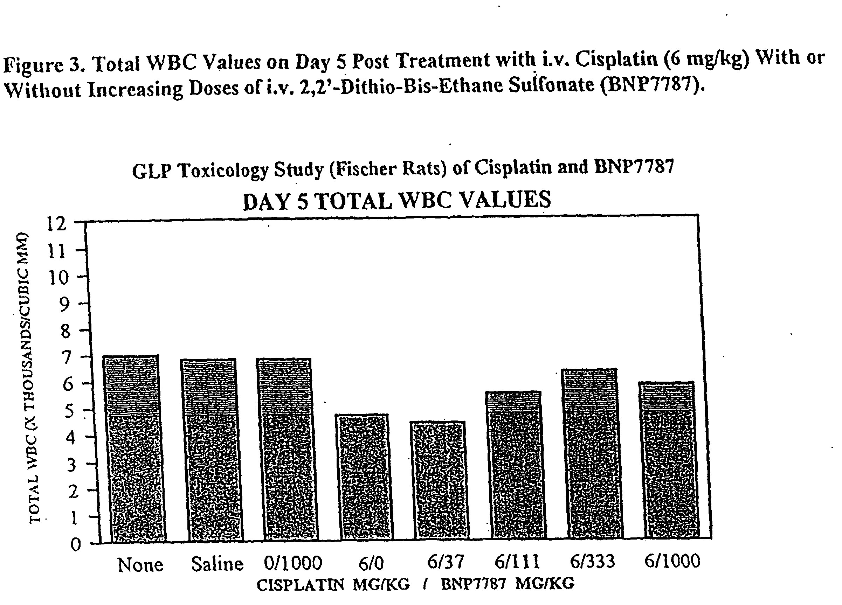

Creatinine (Figure 1), serum blood urea nitrogen (BUN) (Figure 2), each in mg/dl and mean plasma white blood cell counts, in thousands/mm³ (Figure 3) were measured on day 5 and the animals were weighed daily. Figures 1 to 3 display the results in the form of bar charts in which Groups 1 to 8 are arranged in order from left to right. As shown in Figures 1 and 2, BNP7787 demonstrates significant renal protection (100% at 333 and 1000 mg/kg of BNP7787). As shown in Figure 3, the higher (333 and 1000 mg/kg) doses of 2,2'-dithio-bis(ethanesulfonate) gave WBCs of approximately 6,800 and 6,200, respectively. These are within 15% of the WBC values for untreated controls. The day 5 mean WBC counts for the cisplatin-only and low dose (37 mg/kg) 2,2'-dithio-bis-(ethanesulfonate) treatment groups are approximately 4,800 and 4,500, respectively and represent a 32% to 36% reduction from the untreated control group. The Fischer rat model is highly correlated with cisplatin-induced nephrotoxicity in humans.

Thus, this study evidences a dose-dependent effect of 2,2'-dithio-bis(ethanesulfonate) in providing renal protection from cisplatin-induced nephrotoxicity and myelosuppression, including 100% renal protection at higher doses of the sulfonate.

### EXAMPLE 9

### Potentiation of Cisplatin Antitumor Activity by Parenteral Administration of 2,2'-dithio-bis(ethane sulfonate)

The antitumor activity and toxicity, as measured by weight changes estimated by changes in tumor volumes and animal weights, respectively, of escalating doses of intravenously administered cisplatin (6 mg/kg and 9 mg/kg) with or without substantially contemporaneously intravenously administered BNP7787 (1000 mg/kg) was investigated in Fischer rats bearing subcutaneously established (approx. 3.0 g) WARD colon cancer tumors. The WARD tumors will grow in the untreated control rats from 3.0 g to about 10 g in about 7 days.
The results are shown plotted in Figures 4 and 5, where mean body weight (Fig. 4) and median tumor weight (Fig. 5) are plotted on the ordinate against time in days on the abscissa. In both Figures, the key is as follows:
Open circle = untreated, control
Filled circle = BNP 7787, 1000mg/kg
Open inverted triangle = cisplatin (CDDP), 6 mg/kg
Filled inverted triangle = cisplatin, 9 mg/kg
Open square = cisplatin, 6 mg/kg, plus BNP7787, 1000mg/kg
Filled square = cisplatin, 9 mg/kg, plus BNP7787, 1000 mg/kg

As seen in Figure 4, untreated tumor-bearing rats treated with BNP7787 lose only about 2 to 4 % of their body weight in about 6 days, whereas rats treated with cisplatin alone (6 mg/kg and 9 mg/kg) lose up to 8 % of their body weight at 6 days. Treatments with i.v. BNP7787 (1000 mg/kg) at both dose levels of cisplatin (6 mg/kg and 9 mg/kg) were clearly protective against renal damage, as the rats put on more weight than those in all other treatment groups (compare open and closed squares to open and closed triangles). This observation suggests that treatment with BNP7787 may prevent or reduce other cisplatin-associated toxicities, including neurotoxicity and emesis, leading to weight loss in rats.

Moreover, BNP7787 potentiated cisplatin antitumor activity for both the 6 mg/kg and 9 mg/kg dose groups (Figure 5-open and closed squares, respectively). Rats treated with cisplatin only at doses of 6 mg/kg and 9 mg/kg had a maximum reduction in median tumor weight from 3,000 mg to 700 and 500 mg, respectively. Rats treated with cisplatin at doses of 6 mg/kg and 9 mg/kg immediately followed by BNP7787 using a single i.v. dose of 1,000 mg/kg had a maximum reduction in median tumor weight from 3,000 mg to less than 300 and 100 mg, respectively.

## Claims

1. Use of a pharmaceutically acceptable form of 2,2'-dithio-bis(ethanesulfonate) in the manufacture of a medicament for the reduction of the nephrotoxicity of cisplatin, said medicament being for simultaneous or sequential co-administration with cisplatin to a human patient so that the sulfonate and cisplatin become co-present in the blood of the human patient.

2. Use of a pharmaceutically acceptable form of 2,2'-dithio-bis(ethanesulfonate) in the manufacture of a medicament for potentiating the antitumor activity of cisplatin, said medicament being for simultaneous or sequential co-administration with cisplatin to a human patient so that the sulfonate and cisplatin become co-present in the blood of the human patient.

3. Use of a pharmaceutically acceptable form of 2,2'-dithio-bis(ethanesulfonate) in the manufacture of a medicament for potentiating the antitumor activity of cisplatin and reducing the nephrotoxicity of cisplatin, said medicament being for simultaneous or sequential co-administration with cisplatin to a human patient so that the sulfonate and cisplatin become co-present in the blood of the human patient.

4. Use according to claim 1, 2 or 3, wherein the medicament is a sterile solution for intravenous administration.

5. Use according to any of claims 1-4, wherein the medicament is a reconstituted lyophilate of said pharmaceutically acceptable form of 2,2'-dithio-bis(ethanesulfonate).

6. A pharmaceutical composition for use in the treatment of cancer, comprising cisplatin and a pharmaceutically acceptable form of 2,2'-dithio-bis(ethanesulfonate).

7. The composition of claim 6, further comprising a buffer.

8. The composition of claim 6 or 7, which contains chloride anions and sodium and hydrogen cations.

9. The composition of any of claims 6 to 8, which further comprises from 10 to 25 mg/ml of mannitol.

10. The composition of any of claims 6 to 9, which is in the form of a sterile injectable aqueous solution or suspension of pH 2 to 6.

11. The composition of claim 10, wherein the pH is from 4 to 6.

12. The composition of claim 11, which comprises 0.1 to 1.0 mg/ml of cisplatin, 1.0 to 320 mg/ml of the sulfonate, 10 to 25 mg/ml of sodium chloride and hydrochloric or phosphoric acid to provide the defined pH.

13. The composition of any of claims 6 to 9, in the form of a lyophilate.

## Patentansprüche

1. Verwendung einer pharmazeutisch verträglichen Form von 2,2'-Dithiobis(ethansulfonat) bei der Herstellung eines Medikamentes für die Verminderung der Nephrotoxizität von Cisplatin, wobei das Medikament einem Human-Patienten gleichzeitig oder sequentiell zusammen mit Cisplatin verabreicht werden soll, so daß das Sulfonat und Cisplatin zusammen im Blut eines Human-Patienten vorliegen.

2. Verwendung einer pharmazeutisch verträglichen Form von 2,2'-Dithiobis(ethansulfonat) bei der Herstellung eines Medikamentes für die Verstärkung der Antitumorwirkung von Cisplatin, wobei das Medikament einem Human-Patienten gleichzeitig oder sequentiell zusammen mit Cisplatin verabreicht werden soll, so daß das Sulfonat und Cisplatin zusammen im Blut eines Human-Patienten vorliegen.

3. Verwendung einer pharmazeutisch verträglichen Form von 2,2'-Dithiobis(ethansulfonat) bei der Herstellung eines Medikamentes für die Verstärkung der Antitumorwirkung von Cisplatin und die Verminderung der Nephrotoxizität von Cisplatin, wobei das Medikament einem Human-Patienten gleichzeitig oder sequentiell zusammen mit Cisplatin verabreicht werden soll, so daß das Sulfonat und Cisplatin zusammen im Blut eines Human-Patienten vorliegen.

4. Verwendung nach Anspruch 1, 2 oder 3, wobei das Medikament eine sterile Lösung für die intravenöse Verabreichung ist.

5. Verwendung nach einem der Ansprüche 1 bis 4, wobei das Medikament ein zur Infusion hergerichtetes, gefriergetrocknetes Produkt der pharmazeutisch verträglichen Form von 2,2'-Dithiobis(ethansulfonat) ist.

6. Pharmazeutische Zusammensetzung für die Verwendung bei der Behandlung von Krebs, die Cisplatin und eine pharmazeutisch verträglichen Form von 2,2'-Dithiobis(ethansulfonat) umfaßt.

7. Zusammensetzung nach Anspruch 6, die ferner einen Puffer umfaßt.

8. Zusammensetzung nach Anspruch 6 oder 7, die Chloridanionen und Natrium- und Wasserstoffkationen enthält.

9. Zusammensetzung nach einem der Ansprüche 6 bis 8, die ferner 10 bis 25 mg/ml Manitol umfaßt.

10. Zusammensetzung nach einem der Ansprüche 6 bis 9, die in Form einer sterilen injizierbaren wäßrigen Lösung oder Suspension mit pH = 2 - 6 vorliegt.

11. Zusammensetzung nach Anspruch 10, wobei der pH-Wert 4 bis 6 beträgt.

12. Zusammensetzung nach Anspruch 11, die 0,1 bis 1,0 mg/ml Cisplatin, 1,0 bis 320 mg/ml des Sulfonats, 10 bis 25 mg/ml Natriumchlorid und Salzsäure oder Phosphorsäure für die Bereitstellung des definierten pH-Wertes umfaßt.

13. Zusammensetzung nach einem der Ansprüche 6 bis 9 in Form eines gefriergetrockneten Produktes.

## Revendications

1. Utilisation d'une forme acceptable d'un point de vue pharmaceutique du 2,2'-dithio-bis(éthanesulfonate) pour la fabrication d'un médicament destiné à réduire la néphrotoxicité de la cisplatine, ledit médicament étant destiné à la co-administration simultanée ou séquentielle avec la cisplatine à un patient humain de façon à ce que le sulfonate et la cisplatine soient présents simultanément dans le sang du patient humain.

2. Utilisation d'une forme acceptable d'un point de vue pharmaceutique du 2,2'-dithio-bis(éthanesulfonate) pour la fabrication d'un médicament destiné à renforcer l'activité antitumorale de la cisplatine, ledit médicament étant destiné à la co-administration simultanée ou séquentielle avec la cisplatine à un patient humain de façon à ce que le sulfonate et la cisplatine soient présents simultanément dans le sang du patient humain.

3. Utilisation d'une forme acceptable d'un point de vue pharmaceutique du 2,2'-dithio-bis(éthanesulfonate) pour la fabrication d'un médicament destiné à renforcer l'activité antitumorale de la cisplatine et à réduire la néphrotoxicité de celle-ci, ledit médicament étant destiné à la co-administration simultanée ou séquentielle avec la cisplatine à un patient humain de façon à ce que le sulfonate et la cisplatine soient présents simultanément dans le sang du patient humain.

4. Utilisation selon l'une quelconque des revendications 1, 2 ou 3, dans laquelle le médicament est sous la forme d'une solution stérile destinée à l'administration par voie intraveineuse.

5. Utilisation selon l'une quelconque des revendications 1 à 4, dans laquelle le médicament est sous la forme d'un lyophilisat reconstitué de ladite forme acceptable d'un point de vue pharmaceutique du 2,2'-dithio-bis(éthanesulfonate).

6. Composition pharmaceutique destinée à être utilisée dans le traitement du cancer, comprenant la cisplatine et une forme acceptable d'un point de vue pharmaceutique du 2,2'-dithio-bis(éthanesulfonate).

7. Composition selon la revendication 6, comprenant en outre un tampon.

8. Composition selon la revendication 6 ou 7, qui contient des anions chlorure et des cations sodium et hydrogène.

9. Composition selon l'une quelconque des revendications 6 à 8, qui comprend en outre de 10 à 25 mg/ml de mannitol.

10. Composition selon l'une quelconque des revendications 6 à 9, qui est sous la forme d'une solution ou suspension aqueuse injectable stérile ayant un pH compris entre 2 et 6.

11. Composition selon la revendication 10, dans laquelle le pH est compris entre 4 et 6.

12. Composition selon la revendication 11, qui comprend de 0,1 à 1,0 mg/ml de cisplatine, de 1,0 à 320 mg/ml de sulfonate, de 10 à 25 mg/ml de chlorure de sodium et d'acide chlorhydrique ou phosphorique pour conférer le pH défini.

13. Composition selon l'une quelconque des revendications 6 à 9, qui est sous la forme d'un lyophilisat.
